# FASCICULE DE BREVET EUROPEEN

(11) **EP 2 364 135 B1**
(45) Date de publication et mention de la délivrance du brevet: **05.04.2017**
(21) Numéro de dépôt: 09744707.2
(22) Date de dépôt: 06.11.2009
(51) Int. Cl.: A61K 9/00, A61K 9/08, A61K 9/107, A61K 9/12, A61K 31/198, A61P 17/04, A61K 36/54

(54) **MÉDICAMENT OU COMPOSTION DERMATOLOGIQUE COMPRENANT UN EXTRAIT PEPTIDIQUE D'AVOCAT DESTINÉ AU TRAITEMENT ET À LA PRÉVENTION DU PRURIT INTERNE**
ARZNEIMITTEL ODER DERMATOLOGISCHE ZUSAMMENSETZUNG MIT EINEM AVOCADO-PEPTID-EXTRAKT ZUR BEHANDLUNG UND PRÄVENTION VON PRURITUS INTERNE
DRUG OR DERMATOLOGICAL COMPOSITION CONTAINING AN AVOCADO PEPTIDE EXTRACT FOR TREATING AND PREVENTING INTERN PRURITUS

(30) Priorité: 07.11.2008 FR 0857583
(43) Date de publication de la demande: 14.09.2011
(73) Titulaire: Laboratoires Expanscience, 92400 Courbevoie (FR)
(72) Inventeur: MSIKA, Philippe, F-78000 Versailles (FR); BAUDOIN, Caroline, F-78120 Rambouillet (FR); BREDIF, Stéphanie, F-28210 Chaudon (FR)
(74) Mandataire: Regimbeau
(86) Numéro de dépôt international: PCT/EP2009/064785
(87) Numéro de publication internationale: WO 2010/052312

(56) Documents cités:
- WO-A-2007/057439
- WO-A-2008/027533
- US-A- 5 932 230
- US-A1- 2008 113 921
- US-A1- 2008 194 476
- ADEYEMI O O ET AL: "Analgesic and anti-inflammatory effects of the aqueous extract of leaves of Persea americana Mill (Lauraceae)" FITOTERAPIA, IDB HOLDING, MILAN, IT, vol. 73, no. 5, 1 août 2002 (2002-08-01), pages 375-380, XP002318086 ISSN: 0367-326X

## Description

La présente invention concerne un médicament ou composition dermatologique comprenant un extrait peptidique d'avocat destiné au traitement ou à la prévention du prurit interne.

L'utilisation d'extraits peptidiques d'avocat dans le traitement ou la prévention de maladies liées à une déficience du système immunitaire a déjà été décrite dans la demande de brevet WO2005/105123. En effet, il a été démontré que les extraits peptidiques d'avocat sont capables d'augmenter la production de peptides antimicrobiens, en particulier la β-défensine humaine hBD-2. De tels extraits sont donc particulièrement adaptés pour le traitement ou la prévention des peaux enflammées, immatures ou des peaux des personnes immuno-déprimées. Il a également été démontré que de tels extraits peptidiques d'avocat n'induisent pas de réactions inflammatoires, d'irritations ou d'intolérances.

Outre cette action sur les peaux inflammées, immatures ou les peaux des personnes immuno-déprimées, les inventeurs ont maintenant découvert, d'une manière surprenante, qu'une composition comprenant un extrait peptidique d'avocat permet de traiter ou prévenir le prurit interne.

Le prurit est un signe fonctionnel et se définit comme « une sensation qui provoque le besoin de se gratter ». Il peut être localisé, diffus ou généralisé. Il est particulier à la peau et aux demi-muqueuses. Le grattage fait disparaître complètement, mais temporairement, le prurit. Le grattage répétitif, intense, peut entraîner des lésions cutanées. Au sens de la présente invention, on désigne par le terme « prurit » le prurit pathologique qui est gênant pour le patient, parfois même invalidant. Il peut être suffisamment désagréable pour faire préférer au malade la douleur à la démangeaison : les lésions induites par le grattage trouvent là leur explication.

La sensation cutanée désignée couramment par démangeaison peut être considérée comme une réponse uniforme à une grande variété de stimuli physiques et chimiques qui peuvent être de nature endogène ou exogène. Ces stimuli agissent sur les récepteurs des terminaisons nerveuses libres situées à la jonction derme-épiderme et autour des follicules des poils. Le principal agent responsable des démangeaisons est l'histamine, molécule chimique libérée notamment par les mastocytes. L'histamine, lorsqu'elle est libérée, se lie à des récepteurs nerveux de la peau et provoque le prurit. La sensation est transmise par les mêmes fibres nerveuses que celles qui conduisent les sensations douloureuses au cerveau.

Comme l'histamine, la tryptase, protéase elle aussi relarguée par le mastocyte, est également fortement impliquée dans la physiopathologie du prurit : la tryptase induit le signal de prurit en se fixant au récepteur aux protéases (PAR2 ou Proteinase-Activated Receptor 2 ; récepteur 2 activé par la protéinase) au niveau des fibres nerveuses.

La douleur, la température et le toucher sont tous des sensations transmises par les mêmes voies des fibres nerveuses libres non myélinées innervant la peau humaine. Une simulation de faible intensité de ces fibres provoquera du prurit tandis qu'une stimulation plus intense conduira à une réelle sensation de douleur. Bien qu'elle soit transmise par l'intermédiaire des mêmes fibres sensorielles, les impulsions neurales de démangeaison ont une fréquence très inférieure aux impulsions de douleur ; cependant, si un grattage s'ensuit en réponse au stimulus de démangeaison, la fréquence des impulsions neuronales augmente avec l'intensité de grattage et la sensation correspondante vire d'une démangeaison à une douleur.

La démangeaison peut être induite par une grande variété de circonstances, à la fois physiologiques et pathologiques. Notamment lorsqu'elle est généralisée et associée à des lésions cutanées, comme des vésicules et des papules, la démangeaison est souvent l'effet d'une maladie dermatologique primaire et, dans de nombreux cas, elle représente le symptôme fondamental pour le diagnostic. D'un autre côté, en l'absence de lésions cutanées (sur peau saine), la démangeaison peut provenir dans de nombreux cas de désordres systémiques comme, par exemple, des maladies néoplasiques, des désordres métaboliques ou également de réactions allergènes ou d'une hypersensibilité à certains médicaments ou d'un état de grossesse. Enfin, dans d'autres cas, la démangeaison apparaît être le seul symptôme chronique dominant, sans possibilité d'une quelconque clarification sur l'étiopathogénèse de cette affection. Dans ces cas, la démangeaison est normalement désignée par prurit *sine materia,* c'est-à-dire qu'elle représente un symptôme d'origine inconnue, qui n'est pas classifiable en l'absence d'altérations extracutanées ou cutanées identifiables. Les seuls signes objectifs, bien que secondaires, du désordre concerné sont limités à des traces de grattage comme des excoriations linéaires et si la situation dure pendant une durée plus longue, de petites papules secondaires (c'est-à-dire des papules dues au grattage) et des signes de lichenification avec une hyperpigmentation dans les cas plus chroniques.La présente invention a pour objet un extrait peptidique d'avocat,qui comprend 2 à 10% en poids d'azote alpha-aminé par rapport au poids de la matière sèche de l'extrait peptidique, et caractérisé en ce qu'au moins 50% des peptides de l'extrait sont constitués de 10 à 30 acides aminés, pour son utilisation dans le traitement ou la prévention du prurit interne dans un médicament ou une composition dermatologique comprenant ledit extrait et un excipient approprié.

Le terme prurit se subdivise en deux types de prurit : le prurit localisé (tel que les piqûres d'insectes, la dermite séborrhéique, la teigne, les candidoses) et le prurit diffus.

Ce prurit diffus se subdivise lui-même en deux familles : en présence de lésions cutanées spécifiques on parle de dermatoses prurigineuses ; en l'absence de lésions cutanées spécifiques (à l'exception des éventuelles lésions de grattage), on parle de prurits internes.

L'invention se rapporte au traitement et à la prévention de prurits internes. Des tels prurits internes ne sont pas le résultat d'une quelconque dermatose inflammatoire ou d'une déficience du système immunitaire (en particulier de l'immunité innée).

Ainsi, dans le cadre de la présente invention, on envisage le traitement et la prévention du prurit associé à diverses formes de démangeaison dont l'étiologie n'est pas en liaison avec des lésions dermatologiques, en particulier allergiques, (eczéma, urticaire, dermatite atopique, dermatite irritative ou de contact lucites, toxidermie, réaction allergique) ou infectieuses (parasitoses cutanées, dermatophyties, phtiriase du corps et du cuir chevelu, varicelle, VIH, papillonite). En particulier, l'expression « prurit interne » n'englobe pas les dermatites irritatives.

Le prurit est avantageusement choisi dans le groupe constitué par le prurit gravidique, le prurit *sine materia,* le prurit du nouveau-né (lié à la xérose) et le prurit d'origine médicamenteuse. L'invention a donc pour objet un extrait peptidique d'avocat, qui comprend 2 à 10% en poids d'azote alpha-aminé par rapport au poids de la matière sèche de l'extrait peptidique, dans lequel au moins 50% des peptides sont constitués de 10 à 30 acides aminés pour son utilisation dans le traitement ou la prévention du prurit choisi dans le groupe constitué par le prurit gravidique, le prurit *sine materia,* le prurit du nouveau-né (lié à la xérose) et le prurit d'origine médicamenteuse.

Le médicament ou composition dermatologique, qui comprend l'extrait peptidique d'avocat et au moins un excipient, est destiné au traitement et à la prévention du prurit gravidique.

Dix à vingt pour cent des grossesses se compliquent de prurit gravidique. Ce prurit gravidique de la femme enceinte est un prurit diffus sans lésions cutanées spécifiques d'une dermatose inflammatoire ou allergique.

A titre d'exemple de prurit gravidique, on peut plus particulièrement citer la cholestase gravidique. Cette « cholestase du troisième trimestre » est la première cause d'ictère au cours de la grossesse. Les oestrogènes et la progestérone semblent jouer un rôle primordial dans ce prurit. La cholestase gravidique se manifeste par un prurit isolé et intense occasionnant des lésions de grattage et de l'insomnie. Un ictère peut en outre être présent.

L'extrait peptidique d'avocat selon l'invention est également actif dans le traitement du prurigos gravidique, qui serait secondaire à la cholestase gravidique et dont les manifestations sont une éruption de petites papules prurigineuses.

Selon une variante avantageuse de l'invention, l'extrait peptidique d'avocat est destiné au traitement et à la prévention du prurit *sine materia.*

Le groupe de prurit *sine materia* comprend les manifestations de démangeaison apparaissant sans aucune liaison avec des lésions organiques ou avec des maladies systémiques ou de peau (on l'appelle alors prurit psychogène) dans les zones génitales externes (comme le prurit vulvaire et scrotal) et dans la zone périanale (désignée scientifiquement par pruritus ani) ainsi que le prurit de peau âgée (c'est-à-dire le prurit sénile).

Le prurit psychogène (démangeaisons d'origine psychologique) est un prurit relativement fréquent.

On retient à titre de prurit psychogène :
- la parasitophobie où le patient, âgé de plus de 40 ans, se croyant infesté de parasites, procède à des extirpations répétées et mutilantes sur la peau
- la pathomimie qui rend compte de toutes les lésions provoquées ou aggravées par les diverses manoeuvres du patient dont les principales sont les excoriations dues aux ongles.

Le prurit sénile est une affection généralisée très répandue parmi les personnes âgées de 65 ans ou plus. On lui reconnaît une composante psychogène. Le prurit sénile est aggravé par une dessiccation de la peau et une sécheresse cutanée. Une peau déshydratée, généralement combinée à l'exposition aux conditions ambiantes de faible humidité et de température, induit un fin craquage et desquamation de la peau sur les sujets âgés, avec une démangeaison persistante et diffuse en conséquence..

Selon une variante avantageuse de l'invention, l'extrait peptidique d'avocat, est destiné au traitement et à la prévention du prurit du nouveau-né, lié à la xérose du nouveau-né.

La structure de la peau du nouveau-né à terme est identique à celle du nourrisson. Cependant, la peau du nouveau-né à terme présente une particularité qui est la desquamation dite physiologique. Celle-ci affecte 65% des nouveau-nés à terme et représente la plus importante des modifications cutanées néonatales. Elle traduit l'existence d'une xérose cutanée importante. Elle est plus marquée sur les mains, les chevilles et les pieds, mais elle peut être plus étendue. Cette desquamation disparaît spontanément au cours des deux premières semaines de vie.

Les inventeurs ont constaté avec intérêt que l'extrait peptidique d'avocat selon l'invention permet de prévenir ou traiter cette xérose du nouveau-né à terme, en particulier de lutter rapidement (avant la disparition naturelle de cette desquamation) contre la desquamation observée. L'extrait peptidique d'avocat selon l'invention permet également une hydratation et/ou une relipidation de la peau des nouveau-nés et ainsi permet d'accélérer la disparition de la desquamation observée.

Selon une variante avantageuse de l'invention, l'extrait peptidique d'avocat est destiné au traitement et à la prévention du prurit d'origine médicamenteuse.

Le prurit médicamenteux est dû à la prise continue ou intermittente de certains médicaments (barbiturique, antibiotique, aspirine, sulfamide, etc.).

Parmi les molécules susceptibles de provoquer l'apparition d'un prurit, on citera l'acide acétylsalicylique, les antimalariques (chloroquine), les anti-rétroviraux (anti-protéases), les inhibiteurs de l'enzyme de conversion de l'angiotensine, l'interféron, les opiacés (héroïne), les phénothiazines, les rétinoïdes, le tolbutamide et enfin les vitamines du groupe B. L'application sur la peau de substances irritantes à usage cosmétique (par ex. exfoliants) ou professionnel (par ex. laine de verre) peut également provoquer un prurit, que celui-ci s'associe ou non à des lésions cutanées.

Au cours des prurits intenses et chroniques peut également apparaître une lichénification de la peau qui correspond à une forme particulière d'épaississement cutané. Lorsque les lésions de lichénification prennent un aspect nodulaire, on parle de prurigo. Ces lésions de lichénification sont, par ailleurs, elles-mêmes prurigineuses et contribuent de ce fait à l'entretien du prurit. L'extrait peptidique d'avocat selon l'invention est également approprié pour prévenir ou traiter une telle lichénification.

D'une manière tout à fait intéressante, les inventeurs ont constaté que, outre leur effet sur la prévention ou le traitement du prurit, l'extrait peptidique d'avocat présente également une activité relipidante ou hydratante, venant renforcer leur action sur le prurit, en particulier le prurit sénile ou le prurit du nouveau-né.

Le médicament ou la composition dermatologique comprenant l'extrait peptidique selon l'invention est particulièrement adapté pour le traitement des prurits internes, qui peuvent être associés à une sécheresse cutanée ou à une desquamation, chez la femme enceinte, chez le nouveau-né à terme et chez la personne âgée. Avantageusement, le médicament ou la composition dermatologique est indiqué pour la femme enceinte ou pour le nouveau-né à terme.

Dans le cadre de la présente invention, on entend par les termes « azote alpha-aminé », la teneur en azote des peptides sous la forme de groupes alpha-aminés libres. La mesure de la teneur en azote alpha-aminé des peptides permet d'évaluer le degré d'hydrolyse des protéines ainsi que la masse molaire moyenne des peptides.

L'extrait peptidique d'avocat peut directement être obtenu à partir de n'importe quelle partie de l'avocat ou de l'avocatier, telle que le fruit, la peau, le noyau, la feuille ou les racines de l'avocatier. Il est aussi possible d'obtenir un extrait peptidique d'avocat à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile (extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. L'extrait est avantageusement obtenu à partir du fruit frais de l'avocatier. Les fruits pourront être choisis parmi les variétés *Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Red*, plus avantageusement, les variétés *Hass, Fuerte et Reed.* De préférence, on retiendra les variétés *Hass, Fuerte Ettinger et Bacon,* et plus avantageusement les variétés *Hass* et *Fuerte.*

Le fruit de l'avocatier est principalement constitué d'eau, de pulpe, d'huile et de noyau. Les proportions de ces constituants sont, à l'instar de toutes matières naturelles et végétales, extrêmement variables. Toutefois, on admet généralement les données de composition moyennes suivantes, exprimées en pourcentage de fruit frais, données dans le tableau 1 suivant :

**Tableau 1**

| | |
|---|---|
| Eau | 70-85 % |
| Protéines | 1,5-4,5 % |
| Lipides | 12-23 % |
| Sucres | 1,5-5 % |
| Fibres | 1,1-1,6 % |

Par rapport à la pulpe, les protéines de l'avocat représentent 1,5 à 2,5 % (J.P. Gaillard, l'Avocatier, Ed. G.P. Maisonneuve et Larose, 1987, pp 266-67). La répartition en acides aminés, exprimée en pourcentage en poids par rapport au poids total des acides aminés, est donnée dans le tableau 2 suivant :

**Tableau 2**

| | |
|---|---|
| Alanine | 5-7 |
| Arginine | 3-5 |
| Acide aspartique | 8-12 |
| Cystine-cysteine | < 1 |
| Acide glutamique | 11-13 |
| Glycine | 4-6 |
| Histidine | 4-6 |
| Isoleucine | 4-7 |
| Leucine | 8-11 |
| Lysine | 4-7 |
| Méthionine | 1-3 |
| Phénylalanine | 4-6 |
| Proline | 4-7 |
| Sérine | 4-6 |
| Thréonine | 4-6 |
| Tyrosine | 3-6 |
| Valine | 4-7 |

Les principaux acides aminés sont l'acide glutamique, l'acide aspartique et la leucine.

Par comparaison aux plantes oléoprotéagineuses classiques telles que le soja, le tournesol ou le colza, l'avocat est nettement plus pauvre en protéines. Par ailleurs, la relative richesse du fruit en fibres, rend très difficile l'accessibilité à ces protéines par les voies classiques, chimique ou biochimique. De plus, le caractère très peu hydrosoluble de ces macromolécules naturelles invite à préparer des fractions hydrolysées de ces protéines (peptides), présentant une très grande solubilité dans l'eau, et une bien meilleure biodisponibilité. Par ce biais, leur potentiel allergisant peut aussi être éliminé. L'invention aura donc également pour but, la préparation d'un extrait peptidique d'avocat, par une voie de synthèse ménagée et non dénaturante des protéines hydrolysées.

L'extrait peptidique d'avocat est avantageusement un extrait aqueux délipidé. Cet extrait aqueux délipidé subit avantageusement une hydrolyse enzymatique. Les protéines d'avocat ont avantageusement été hydrolysées par des protéases. Selon une variante avantageuse de l'invention, les sucres présents dans l'extrait aqueux délipidé sont également hydrolysés (en présence de glucanases par exemple).

Plus particulièrement, l'extrait peptidique d'avocat est susceptible d'être obtenu par un procédé comprenant les étapes suivantes :
- obtention d'un tourteau d'avocat, avantageusement du fruit de l'avocat, par séchage puis extraction de l'huile (lipides); ensuite
- cryobroyage et délipidation complète dudit tourteau puis décantation, centrifugation et récupération du gâteau ; puis
- première hydrolyse en présence de glucanases, suivie d'une centrifugation et de l'élimination de la fraction soluble ;
- seconde hydrolyse en présence d'une ou plusieurs protéases, suivie d'une centrifugation et de l'élimination du culot ; ensuite
- concentration de la phase peptidique par nanofiltration ;
- décoloration, en présence de charbon actif par exemple, suivie d'une filtration simple (10 µm) puis d'une ultrafiltration (seuil de coupure de 10 kD) ; enfin
- le cas échéant, micro filtration stérilisante finale (0,2 µm), ajout de conservateur et conditionnement.

Selon une variante avantageuse de l'invention, la première étape du procédé consiste à sécher le fruit puis à le déshuiler. Ainsi, après tranchage du fruit en fines lamelles, son séchage peut être réalisé par l'ensemble des techniques connues de l'homme de métier, parmi lesquelles on peut citer le séchage à l'air chaud, la lyophilisation ou encore le séchage osmotique. D'une façon générale, la température lors de cette étape de séchage sera avantageusement maintenue, quelle que soit la technique employée, inférieure ou égale à 80°C. Dans le cadre du présent procédé, pour des raisons de facilité de mise en oeuvre et pour des raisons de coût, le séchage en séchoirs ventilés, en couche mince et sous courant d'air chaud, à une température comprise entre 70 et 75°C est préféré. La durée de l'opération peut varier de 5 à 72 heures.

Les lipides du fruit séché sont par la suite extraits soit par voie mécanique dans une presse à vis continue ou encore par voie chimique, à l'aide d'un solvant tel que l'hexane dans un extracteur de type Soxhlet ou dans un extracteur continu à bande, de type De Smet®, notamment selon le procédé décrit dans la demande FR 2 843 027, ou encore par un procédé utilisant le CO₂ supercritique. Parmi les intérêts majeurs du procédé, l'huile co-produite constitue un produit bien évidemment directement valorisable. C'est pourquoi, on préfère l'extraction des lipides par voie mécanique.

Le fruit sec et déshuilé, ou encore appelé tourteau peut subir ensuite les étapes suivantes :
- cryobroyage,
- délipidation complète, notamment par un solvant alimentaire non toxique tel que l'éthanol et/ou l'acétone,
- décantation, puis lavage du tourteau à l'eau,
- centrifugation, et récupération du gâteau,
- première hydrolyse en présence d'une ou plusieurs glucanases,
- centrifugation et élimination de la fraction soluble,
- seconde hydrolyse en présence d'une ou plusieurs protéases,
- centrifugation et élimination du culot,
- concentration par nanofiltration,
- décoloration en présence de charbon actif,
- filtration simple (10 µm) puis ultrafiltration (seuil de coupure de 10 kD),
- ajout de conservateur, microfiltration stérilisante finale (0,2 µm) et conditionnement.

L'extrait aqueux final peut contenir en poids 1 à 60 % de matière sèche, ou encore 3 à 20 % de matière sèche, de préférence 5 à 6 % de matière sèche. Par rapport au poids de la matière sèche, la teneur massique en azote alpha-aminé pourra être comprise entre 2 et 10 %, de préférence entre 5 et 7 %. Le pH d'une solution aqueuse à 1,2 % en poids d'extrait sec, sera généralement compris entre 3 et 6, plus avantageusement entre 4 et 5. Les données analytiques moyennes d'une solution aqueuse à 1,2 % en poids d'extrait sec, obtenue par le procédé décrit précédemment, sont données dans le tableau 3 suivant :

**Tableau 3**

| | | |
|---|---|---|
| Azote α-aminé (méthode dite « o-phthalaldéhyde » ou « ninhydrine ») (en pourcentage massique dans la matière sèche) | | 4 - 10 |
| Protéines (en pourcentage massique dans la matière sèche) (N x 6,25)¹ | | 10 - 30 |
| pH (dilution ¼) | | 4,5 - 7,0 |
| Absorbance (dilution ¼) | 420 nm | 0,1 - 0,6 |
| | 550 nm | 0,02 - 0,1 |

| | | |
|---|---|---|
| ¹ N x 6,25 correspond au dosage de l'azote total (N) d'un échantillon multiplié par un coefficient spécifique pour la protéine dosée. Quand on ne connaît pas précisément le coefficient des protéines dosées, on utilise par convention le coefficient 6,25. | | |

Le tableau 4 suivant donne la composition moyenne en acides aminés de l'extrait peptidique obtenu par le procédé selon l'invention, dans lequel les valeurs sont exprimées en pourcentage en poids par rapport au poids total des acides aminés dosés. Les valeurs pour l'acide aspartique et l'acide glutamique incluent également les teneurs en asparagine et en glutamine, respectivement.

**Tableau 4**

| Acide aminé | Valeur minimale | Valeur maximale |
|---|---|---|
| Alanine | 6,4 | 7,8 |
| Arginine | 4,7 | 5,7 |
| Acide aspartique | 10,3 | 12,7 |
| Cystine-cystéine | 2,9 | 3,5 |
| Acide glutamique | 13,0 | 15,8 |
| Glycine | 5,3 | 6,5 |
| Histidine | 2,2 | 2,6 |
| Isoleucine | 4,8 | 5,8 |
| Leucine | 7,6 | 9,4 |
| Lysine | 3,0 | 3,8 |
| Méthionine | 1,2 | 1,6 |
| Phénylalanine | 4,7 | 5,7 |
| Proline | 4,1 | 5,2 |
| Sérine | 5,5 | 6,7 |
| Thréonine | 4,6 | 5,6 |
| Tyrosine | 3,6 | 4,4 |
| Valine | 5,8 | 7,2 |

| | | |
|---|---|---|
| Tryptophane non dosé | | |

L'extrait obtenu pourra éventuellement être lyophilisé dans le but d'obtenir une poudre solide (extrait sec), mais totalement hydrosoluble par rapport aux protéines originelles de l'avocat.

50 % au moins des peptides de l'extrait sont constitués de 10 à 30 motifs d'acides aminés. La taille de ces peptides est donc beaucoup plus petite par comparaison à celle des protéines natives de l'avocat. Ces peptides présentent donc à ce titre une bien meilleure bio disponibilité, notamment cutanée.

Selon une variante avantageuse de l'invention, le médicament ou composition dermatologique comprend 0,01 à 20% en poids sec d'extrait peptidique d'avocat, par rapport au poids total dudit médicament ou composition dermatologique, encore plus avantageusement 0,1 à 15% en poids sec d'extrait peptidique d'avocat, encore plus avantageusement 0,5 à 10% en poids sec d'extrait peptidique d'avocat, encore plus avantageusement 0,7 à 8% en poids sec d'extrait peptidique d'avocat, et encore plus avantageusement 1 à 5% en poids sec d'extrait peptidique d'avocat.

Le médicament ou composition dermatologique peut en outre comprendre au moins un composé choisi dans le groupe constitué par les agents anti-irritant et/ou apaisant et/ou cicatrisant et/ou anti-vieillissement et/ou hydratant.

Les agents anti-irritants et/ou apaisant et/ou cicatrisant et/ou anti-vieillissement et/ou hydratant pouvant être utilisés dans le cadre de la présente invention en association avec l'extrait peptidique d'avocat sont avantageusement la glycine, les sucres et/ou peptides végétaux dont le lupin, le lupéol (FR 2 822 821, FR 2 857 596), les oxazolines (WO 03/055463), les oxazolidinones (WO 2004/050052), l'acide lipoïque, l'alpha bisabolol, les dérivés de réglisse, l'enoxolone, l'ectoïne, des furanes d'avocat (en particulier l'Avocadofurane® : furanes d'avocat, pouvant être obtenus par le procédé décrit dans la demande internationale WO 01/21605), les extraits de Centella asiatica en particulier l'acide madécassique ou l'acide asiatique, la caféine, le rétinol, le rétinal, l'acide rétinoïque, l'oxyde de zinc, le magnésium, le silicium, le cuivre, le zinc, le manganèse, le sélénium, l'acide hyaluronique, l'acide azélaïque et ses sels ou esters, l'acide salicylique et ses dérivés, l'alpha hydroxyacide (AHA), les esters d'AHA, l'acide pyrrolidone carboxylique et dérivés, les céramides, le cholestérol, le squalane, les phospholipides, le beta carotène, la vitamine A, la vitamine E, la vitamine C, la vitamine B3 (niacinamide, nicotinamide), la vitamine B5 (panthenol), la vitamine B6, le peroxyde de benzoyle, l'urée, la coenzyme Q10, la glucosamine et ses sels, la N-acétyl glucosamine, les eaux de source ou thermales (eau d'Avène, eau de la Roche Posay, eau de Saint Gervais, eau d'Uriage, eau de Gamarde), les peptides de soja et l'arabinogalactane (en particulier l'arabinogalactane peut être associé à du lupéol ou des peptides de soja).

Les oxazolines pouvant être utilisées dans le cadre de la présente invention en association avec l'extrait peptidique d'avocat sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide^{®}.

Outre ces actifs, l'extrait peptidique d'avocat selon l'invention, seul ou en association avec les actifs précédemment cités, peut être utilisé en association des actifs protecteurs solaires, tels que des filtres ou écrans solaires UVB et/ou UVA ; tels les écrans ou filtres minéraux et/ou organiques connus de l'homme du métier qui adaptera leur choix et leurs concentrations en fonction du degré de protection recherché.

A titre d'exemple d'actifs protecteur solaire, on peut notamment citer l'oxyde de titane, l'oxyde de zinc, le méthylène bis-benzotriazolyl tétraméthylbutylphénol (nom commercial : TINOSORB M) et le Bis-éthylhéxyloxyphénol méthoxyphényl triazine (nom commercial : TINOSORB S).

Outre ces actifs, l'extrait peptidique d'avocat selon l'invention, seul ou en association avec les actifs précédemment cités, peut être utilisé en association avec les sucres d'avocat, des agents restructurant de la barrière cutanée, des composés antifongiques, des conservateurs antiseptiques et des composés contenant des insaponifiables d'huiles végétales, les peptides de lupin, l'huile d'avocat, le butyl avocadate, les cyclocéramides, la génistéine, les concentrats de colza, les concentrats de maïs.

Les sucres d'avocats sont avantageusement le D-mannoheptulose et/ou le perséitol. Les sucres d'avocats correspondent plus avantageusement à l'extrait hydrosoluble de sucres d'avocat décrit dans la demande WO 2005/115421.

Les agents restructurant de la barrière cutanée, permettant de stimuler la synthèse des lipides clés de l'épiderme, et pouvant être utilisés dans le cadre de la présente invention en association avec l'extrait peptidique d'avocat sont avantageusement des concentrats de tournesol, encore plus avantageusement des concentrats de tournesol linoléiques, tels que l'actif commercialisé par les Laboratoires Expanscience, Soline^{®} (cf. la demande internationale WO 01/21150), des insaponifiables d'huile végétale, tel que l'Avocadofurane^{®} (cf. la demande internationale WO 01/21150), des agonistes PPARs (rosiglitazone, pioglitazone).

Les composés antifongiques pouvant être utilisés dans le cadre de la présente invention en association avec l'extrait peptidique d'avocat sont avantageusement l'econazole et le ketoconazole.

Les conservateurs antiseptiques pouvant être utilisés dans le cadre de la présente invention en association avec l'extrait peptidique d'avocat sont par exemple le triclosan, la chlorhéxidine, les ammoniums quaternaires.

Les composés contenant des insaponifiables d'huiles végétales pouvant être utilisés dans le cadre de la présente invention en association avec l'extrait peptidique d'avocat sont avantageusement choisis dans le groupe constitué par les lipides furaniques d'avocat, les insaponifiables d'avocat, les insaponifiables de soja, les insaponifiables d'avocat et de soja, les concentrats d'huile de lupin, les concentrats d'huile de tournesol et leurs mélanges.

Les lipides furaniques d'avocat pouvant être utilisés dans le cadre de la présente invention en association avec l'extrait peptidique d'avocat sont avantageusement des 2-alkyl furanes naturels, notamment l'actif Avocadofurane^{®} commercialisé par les Laboratoires Expanscience, pouvant être obtenus par le procédé décrit dans la demande internationale WO 01/21605.

Les insaponifiables d'avocat et de soja pouvant être utilisés dans le cadre de la présente invention en association avec l'extrait peptidique d'avocat sont avantageusement un mélange d'insaponifiables d'avocat furanique et d'insaponifiables de soja, dans un rapport respectif d'environ 1/3-2/3. Les insaponifiables d'avocat et de soja sont encore plus avantageusement le produit Piasclédine^{®}, commercialisé par les Laboratoires Expanscience.

Les concentrats d'huile de lupin pouvant être utilisés dans le cadre de la présente invention en association avec l'extrait peptidique d'avocat sont avantageusement des concentrats obtenus par distillation moléculaire d'huile de lupin, avantageusement d'huile de lupin blanc doux, tels que ceux décrits dans la demande internationale WO 98/47479. Ils contiennent avantageusement environ 60% en poids d'insaponifiables.

Les concentrats d'huile de tournesol pouvant être utilisés dans le cadre de la présente invention en association avec l'extrait peptidique d'avocat sont avantageusement des concentrats de tournesol linoléiques, tels que l'actif commercialisé par les Laboratoires Expanscience, Soline^{®} (cf. la demande internationale WO 01/21150).

Le médicament ou la composition dermatologique peut être formulé sous la forme de différentes préparations adaptées à une administration topique. De préférence les différentes préparations incluent les crèmes, les laits, les lotions, les eaux, les huiles, les patches, les sprays ou tout autre produit pour application externe.

Les modes d'administration, les posologies et les formes galéniques optimales des composés et compositions peuvent être déterminés selon les critères généralement pris en compte dans l'établissement d'un traitement pharmaceutique, en particulier dermatologique, adapté à un patient comme par exemple l'âge ou le poids corporel du patient, la gravité de son état général, la tolérance au traitement, les effets secondaires constatés, le type de peau. Le patient est avantageusement une femme enceinte, un nouveau-né à terme ou une personne âgée, plus avantageusement une femme enceinte ou un nouveau-né à terme.

En fonction du type d'administration souhaitée, le médicament et/ou les composés actifs peuvent en outre comprendre au moins un excipient pharmaceutiquement acceptable, notamment dermatologiquement acceptable. De préférence, on utilise un excipient adapté pour une administration par voie topique externe. Le médicament ou composition dermatologique peut en outre comprendre au moins un adjuvant pharmaceutiquement connu de l'homme du métier, choisi parmi les épaississants, les conservateurs, les parfums, les colorants, des filtres chimiques ou minéraux, les agents hydratants, les eaux thermales, etc.

Au vu des patients visés, en particulier femme enceinte et nouveau-né à terme, le médicament ou la composition dermatologique ne comprendra pas d'excipients à effet notoire, tels que des allergènes, des savons contenant de l'iode, de l'alcool benzylique, de vecteurs de pénétration (glycol) et des composés Cancérigènes Mutagènes et Reprotoxiques de classe 1,2,3. En outre, les parfums éventuellement contenus dans la formulation seront avantageusement des parfums neutres.

Les exemples suivants permettent d'illustrer l'invention, ils ne sont pas limitatifs.

### Exemple 1 : préparation d'un extrait peptidique d'avocat

50 kg d'avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80°C, sa durée est quant à elle de 48 heures. Une fois séchés, les fruits sont soumis à une pression à froid. Cette opération est réalisée sur une petite presse Komet® de laboratoire.

Les 4 kg de fruits délipidés (tourteau) sont alors broyés à froid puis extraits à reflux, en présence de 25 litres d'éthanol. La poudre épuisée en lipides est alors récupérée par filtration sur Büchner et séchée à l'étuve à 50°C, pendant 5 heures.

Le tourteau est alors lavé à l'eau déminéralisée (10 litres) puis séparé par centrifugation. La fraction solide est reprise dans une solution aqueuse, acidifiée par HCl (pH fixé à 5), puis mise en présence de 2 % de cellulase par rapport à la matière sèche. La durée d'hydrolyse est fixée à 6 heures.

Le mélange est ensuite centrifugé à 2000 g en présence d'adjuvant (2,5% p/v). Le culot récupéré subit alors une seconde hydrolyse à pH 8,0, en présence de 0,5 % de protéase, à une température de 55°C, pendant 2 heures. L'hydrolyse est régulée à pH constant par ajout continu de soude 2M. La protéase est enfin dénaturée par chauffage pendant 10 minutes, à 85°C.

Le mélange obtenu est centrifugé et le surnageant filtré par passage à travers une membrane de 7,5 µm. Il est ensuite ultrafiltré sur des membranes présentant un seuil de coupure de 10 kD.

L'extrait peptidique brut obtenu à 20 % de matière sèche, est décoloré en présence de 1% de charbon actif, puis filtré à nouveau à travers une membrane de 7,5 µm. L'extrait décoloré est alors microfiltré (0,2 µm), ajusté en titre à hauteur de 5 % de matière sèche, puis complémenté en conservateur et enfin conditionné après une filtration stérilisante (0,2 µm).

Les caractéristiques de l'extrait peptidique d'avocat hydrosoluble à 5 % de matière sèche, obtenu par ce procédé sont données dans le tableau 5 suivant :

**Tableau 5**

| Aspect | Solution de couleur légèrement orangée |
|---|---|
| **Critères analytiques** | |
| Matière sèche | 5% |
| pH (dilution ¼) | 4,5 |
| Absorbance à 420 nm (dilution ¼) | 0,152 |
| Absorbance à 550 nm (dilution ¼) | 0,035 |

| **Composition de la matière sèche** | |
|---|---|
| Azote alpha-aminé | 6,7% |
| Protéines | Non détectées |
| Conservateur | 0,4 % |

Dans le tableau 6 suivant, on donne la répartition des masses moléculaires dans l'extrait peptidique d'avocat obtenu par ce procédé :

**Tableau 6**

| Pic HPLC | Masse molaire (g/mol) | Nombre d'acides aminés moyen | % relatif |
|---|---|---|---|
| 1 | > 3480 (1) | < 29 | 1 % |
| 2 | 3480 à 1180 | 29 à 9 | 26 % |
| 3 | 1180 à 310 | 9 à 2 | 45 % |
| 4 | 310 à 130 | 2 à 1 | 15 % |
| 5 | < 130 | 1 | 13 % |

| | | | |
|---|---|---|---|
| (1) masses < 10 000 g/mol. | | | |

On constate que 27 % au moins des peptides de l'extrait sont constitués d'au moins 9 motifs d'acides aminés. Par conséquent, la taille des peptides de l'extrait est donc extrêmement petite par comparaison à celle des protéines natives de l'avocat. Ces peptides présentent donc à ce titre une bien meilleure bio disponibilité, notamment cutanée.

### Exemple 2 : extrait peptidique d'avocat : activités sur le prurit

Le prurit est défini comme une sensation cutanée déplaisante qui provoque le désir de se gratter. Le « message » de prurit est transmis par les terminaisons libres des fibres C.

Le mastocyte semble jouer un rôle fondamental dans les prurits d'origine cutanée. En effet, de nombreuses substances prurigineuses provoquent sa dégranulation. Lors de sa dégranulation, le mastocyte excrète divers produits, parmi lesquels :
- l'histamine dont la libération provoque l'activation des récepteurs H1 au niveau des fibres nerveuses et donc l'induction du signal de prurit,
- les protéases, et en particulier la tryptase, qui induisent le signal de prurit en se fixant au récepteur aux protéases (PAR2 ou Proteinase-Activated Receptor 2 ; récepteur 2 activé par la protéinase) au niveau des fibres nerveuses.

Le relargage des médiateurs prurigineux par le mastocyte aboutissant à la transmission du signal nerveux de prurit a également d'autres conséquences qui vont amplifier le phénomène :
- Ainsi, l'activation des récepteurs PAR2 par la tryptase au niveau des fibres nerveuses entraîne un relargage par ces fibres de neuropeptides, comme la substance P et le CGRP (calcitonine gene related peptide ou peptide relatif au gène de la calcitonine). La substance P, à son tour, est capable d'induire la dégranulation du mastocyte. CGRP et substance P agissent également sur les cellules endothéliales induisant ainsi une vasodilatation, extravasation et donc recrutement de cellules de l'inflammation ;
- L'histamine est, elle aussi, capable de stimuler la libération par les nerfs sensitifs de neuromédiateurs, tels que la substance P et la neurokinine A qui peuvent entraîner la dégranulation du mastocyte (et donc une amplification de la réaction) ;
- La tryptase peut également activer et induire la dégranulation du mastocyte qui possède également des récepteurs PAR2 ;
- De même, les cellules endothéliales peuvent répondre à une stimulation par la tryptase *via* le récepteur PAR2 ;
- Par ailleurs, les cellules endothéliales peuvent également répondre à une stimulation par l'histamine ;
- Enfin, le PAR2 est également exprimé par les kératinocytes épidermiques dans lesquels il est impliqué dans la mise en place d'une réaction inflammatoire *via* l'activation du facteur de transcription NFκB.

Les études présentées ci-après ont été réalisées sur des mastocytes péritonéaux de rat. Ces cellules présentent les mêmes caractères que les mastocytes cutanés et constituent à ce titre un modèle expérimental représentatif de la peau humaine.

### I. Effet de l'extrait peptidique d'avocat sur la libération de tryptase par des mastocytes

### I.1. Matériel et Méthodes :

Des mastocytes péritonéaux de rat (à raison de 2.10⁵ cellules / ml) ont été incubés pendant 30 minutes à 37°C en présence, ou non, de l'extrait peptidique d'avocat (obtenu à l'exemple 1) à 0,005% (p/v) ou de la quercétine (Sigma) à 50µM, inhibiteur de référence de la libération de tryptase. Les cellules ont été ensuite stimulées pendant 15 minutes par le calcium ionophore A23187 (Millipore) à 0,5µM. Le calcium ionophore est utilisé comme inducteur de la libération de tryptase (il induit une entrée et une mobilisation du calcium intracellulaire). La réaction a été stoppée par centrifugation à 4°C.

La libération de tryptase a été évaluée par dosage de la tryptase dans les culots cellulaires et dans les surnageants au moyen du kit « Mast cell degranulation assay kit » (Millipore). Ce test est basé sur la détection spectrophotométrique du chromophore p-nitroaniline (pNA) après clivage du substrat tosyl-gly-pro-lys-pNA par la tryptase.

L'absence de cytotoxicité de l'extrait peptidique d'avocat dans les conditions de l'essai a été confirmée par une coloration au bleu Trypan.

### I.2. Résultats :

Comme le montre le tableau 7, le calcium ionophore A23187 a induit une libération massive de tryptase et la quercétine à 50 µM a significativement inhibé cette libération (-47%, p<0,01) ; ces conditions valident l'essai.

**Tableau 7 : Libération de tryptase par des mastocytes**

| | Tryptase (ng/ml) | | |
|---|---|---|---|
| | Moyenne | Ecart-type | Pourcentage d'inhibition |
| Témoin non stimulé | 2 | 1 | |
| Témoin stimulé (Ca Ionophore) | 83 | 5 | |
| Quercétine 50 µM | 44 | 2 | -47% ** |
| Extrait Peptidique d'Avocat 0,005% | 58 | 7 | -30% ** |

| | | | |
|---|---|---|---|
| ** p<0,01 - test t de Student | | | |

L'extrait peptidique d'avocat à la concentration de 0,005% (p/v) a significativement inhibé (-30%) la libération de tryptase par des mastocytes induite par le calcium ionophore.

### II. Effet de l'extrait peptidique d'avocat sur la libération d'histamine par des mastocytes

### II.1. Matériel et Méthodes :

Des mastocytes péritonéaux de rat (en suspension à raison de 10 000 cellules / 250 µl de tampon Tyrode) ont été incubés pendant 30 minutes à 37°C en présence, ou non, de l'extrait peptidique d'avocat, obtenu à l'exemple 1, à 0,05%, 0,5% et 1% (p/v) ou de Calcium à 10 mM, utilisé comme référence inhibitrice de la libération d'histamine.

Les cellules ont ensuite été stimulées pendant 10 minutes par la substance P à 10 µM (Neosystem) en présence de 0,3 mM de calcium.

La réaction a été stoppée par centrifugation, les surnageants et culots cellulaires ont été repris avec de l'acide perchlorique 0,8N afin de réaliser le dosage de l'histamine par méthode spectrofluorométrique.

Les quantités d'histamine libérée ont été exprimées en pourcentage de libération : quantité dans les surnageants / quantité totale.

L'absence de cytotoxicité de l'extrait peptidique d'avocat dans les conditions de l'essai a été confirmée par un dosage de LDH (Lactate Déshydrogénase).

### II.2. Résultats :

Comme le montre le tableau 8, la libération d'histamine a été fortement stimulée par la substance P et le Calcium à 10 mM a significativement inhibé (-78%) cette libération ; ces conditions valident l'essai.

**Tableau 8 : Libération d'histamine par des mastocytes**

| | Histamine (% libération) | | |
|---|---|---|---|
| | Moyenne | Ecart-type | Pourcentage d'inhibition |
| Témoin non stimulé | 2 | 1 | |
| Témoin stimulé (Substance P) | 74 | 5 | |
| Calcium 10 mM | 16 | 1 | -78% ** |
| Extrait Peptidique d'Avocat 0,05% | 58 | 5 | -21% * |
| Extrait Peptidique d'Avocat 0,5% | 60 | 4 | -19% * |
| Extrait Peptidique d'Avocat 1% | 35 | 2 | -53%** |

| | | | |
|---|---|---|---|
| * p<0,05 ; ** p<0,01 - test t de Student | | | |

L'extrait peptidique d'avocat aux concentrations de 0,05%, 0,5% et 1% (p/v) a entraîné une inhibition de la libération d'histamine induite par la substance P significative et dose dépendante (respectivement -21%, -19% et -53%).

### III. Effet de l'extrait peptidique d'avocat sur l'expression génique du PAR2 dans des épidermes reconstruits

### III.1. Matériel et Méthodes :

Des épidermes reconstruits Skinethic de 17 jours ont été traités en systémique par l'extrait peptidique d'avocat (obtenu à l'exemple 1) à 0,04% (p/v) ou par l'acide rétinoïque [Sigma] à 1 µM (référence positive) pendant 6h.

L'analyse de l'expression des gènes a été réalisée en utilisant des DNA arrays simplifiés standards. Ces mini-chips ont été réalisées sur support Nylon, en spottant des cDNA spécifiques des marqueurs d'intérêt. L'analyse a été réalisée selon une technologie miniaturisée et optimisée propre, basée sur l'utilisation d'ARN messager et d'un marquage au 33P.

A la fin de la culture, les épidermes ont été rincés avec une solution de PBS (phosphate buffered saline ou tampon phosphate salin). L'ARN total des épidermes a ensuite été extrait et purifié à l'aide du Tri-Reagent [Sigma] selon un protocole standardisé. La quantité et la qualité des ARNs ont ensuite été évaluées à l'aide d'un bioanalyseur Agilent 2001. L'ARNm de chaque culture a été réverse-transcrit en utilisant de l'oligodT et un déoxyribonucléotide triphosphate marqué au 33P. Des séquences marquées « cibles » cDNA multiples ont été réalisées pour chaque culture. Ces cibles ont ensuite été hybridées, dans des conditions optimisées, aux cDNA « sondes » en excès fixés sur les membranes. Après lavage, la quantité relative de cible marquée a été révélée par comptage direct sur PhosphorImager.

L'analyse des membranes a été réalisée à l'aide d'un logiciel approprié. Les données traitées mettent en évidence l'expression différentielle des différents gènes suite au traitement par l'extrait peptidique d'avocat en comparaison avec le témoin correspondant.

### III.2. Résultats :

Comme le montre le tableau 9, l'expression génique du PAR2 dans des épidermes reconstruits a été inhibée (-29%) par l'acide rétinoïque, témoin de l'essai.

**Tableau 9 : Expression génique de PAR2 dans des épidermes reconstruits**

| PAR2 (Expression relative) | | | |
|---|---|---|---|
| | Moyenne | Ecart-type | Pourcentage d'inhibition |
| Epidermes contrôles | 4,2 | 0,1 | |
| Acide rétinoïque 1 µM | 3 | 0,3 | - 29% |
| Extrait Peptidique d'Avocat 0,04% | 2 | 0 | -52% |

L'extrait peptidique d'avocat, à la concentration de 0,04% (p/v) a induit une inhibition de l'expression génique du PAR2 (-52%).

### IV. Conclusion

Nous avons pu montrer que l'extrait peptidique d'avocat est capable de moduler le relargage d'histamine et de tryptase par des mastocytes. Ainsi, l'extrait peptidique d'avocat a un effet inhibiteur sur la dégranulation du mastocyte à l'origine de l'induction et de l'entretien de la réaction de prurit.

Par ailleurs, nous avons montré un effet inhibiteur de l'extrait peptidique d'avocat sur l'expression épidermique du récepteur PAR2 (Proteinase Activated Receptor 2).

L'extrait peptidique peut donc prévenir les effets de l'activation du PAR2 dans la mise en place du prurit; plus particulièrement l'extrait peptidique d'avocat peut moduler les effets de la tryptase sur ses différentes cibles cellulaires, la tryptase étant un agoniste endogène du PAR2.

### Exemple 3 : extrait peptidique d'avocat : activités hydratante et relipidante

L'hydratation naturelle de la peau est principalement due à son contenu en glycosaminoglycanes et en particulier à l'acide hyaluronique.

Les glycosaminoglycanes (GAGs) autres que l'acide hyaluronique sont des molécules composées d'une partie peptidique et d'une partie osidique. Ils sont fortement impliqués dans la rétention d'eau dans le derme et contribuent ainsi au maintien d'un taux d'hydratation convenable.

L'acide hyaluronique (AH) est composé exclusivement de molécules osidiques. Il est notamment un facteur important de l'épaississement de l'épiderme : il forme avec l'eau, à laquelle il se fixe fortement, un réseau visco-élastique dense et contribue ainsi à la densité, à la cohésion, à l'hydratation et à l'élasticité de la peau. Avec l'âge, il y a une diminution du contenu en AH de l'épiderme.

La synthèse de GAGs sulfatés et de l'AH a été étudiée sur kératinocytes en culture suite à un traitement par l'extrait peptidique d'avocat, selon l'exemple 1, afin d'évaluer l'activité hydratante de ce dernier.

L'une des principales fonctions de la peau est celle de barrière de protection contre les agressions de l'environnement et contre les pertes en eau.

La barrière perméable de la peau se situe dans la couche externe de l'épiderme : le *stratum corneum.* Les corneocytes (kératinocytes dans un état de différenciation avancé), cellules constitutives du *stratum corneum*, sont enrobés dans une matrice extra-cellulaire enrichie en lipides. C'est cette matrice riche en lipides qui est à l'origine de la fonction de barrière perméable.

Les lipides du *stratus corneum* sont principalement constitués de céramides (50% de la masse totale des lipides), de cholesterol (25%) et d'acides gras libres (10%).

Le contenu en ces trois lipides clés du *stratum corneum* est réduit dans le cas de xérose, cette diminution viendrait d'une baisse de l'activité des enzymes limitantes des voies de synthèse de ces lipides.

L'effet de l'extrait peptidique d'avocat sur la néosynthèse des lipides épidermiques a été évalué dans un modèle d'épiderme reconstruit, avec analyse séparée des phospholipides (membranes cellulaires) et des lipides neutres (incluant les lipides « spéciaux » de l'épiderme).

### I. Effet sur la synthèse des glycosaminoglycanes et de l'acide hyaluronique par des kératinocytes (Activité hydratante)

### I.1 Matériel et méthode :

Des kératinocytes épidermiques humains normaux (NHEK) ont été pré-cultivés pendant 24 heures à 37°C, 5% de CO2 dans du milieu SFM [Invitrogen] pauvre en calcium (0,09 mM) supplémenté en EGF 0,25 ng/ml [Invitrogen], extrait pituitaire 25 µg/ml [Invitrogen] et gentamycine 25 µg/ml [Sigma].

Les kératinocytes ont ensuite été traités pendant 72 heures par l'extrait peptidique d'avocat, selon l'exemple 1, à 0,05% MS et 0,005% MS ou par les références : acide rétinoïque 0.1µM [Sigma] ou calcium 1,5 mM [Prolabo]. Deux séries identiques ont été préparées afin de doser d'une part la libération/production d'acide hyaluronique et d'autre part l'incorporation de sulfure dans la fraction GAGs. Le lot correspondant au dosage des GAGs a été supplémenté par du 35S-sulfate [Amersham] après 48 heures de culture (marquage pendant les 24 dernières heures).

A la fin du traitement, dans le lot correspondant au dosage des GAGs, la radioactivité incorporée a été comptée et ainsi, la variation de la synthèse des GAGs par rapport au contrôle analysée.

Dans le lot correspondant au dosage de l'acide hyaluronique, à la fin du traitement, les surnageants de culture ont été prélevés et l'acide hyaluronique dosé à l'aide d'un test ELISA modifié spécifique [Biogenic].

Les comparaisons intergroupes ont été réalisées par analyse de variance à l'aide du test de comparaison multiple de Dunnett.

### I.2 Résultats :

### I.2.1 Effet sur la synthèse des GAGs :

Le calcium, utilisé comme référence (effet pro-différenciant) stimule l'incorporation de 35S-sulfate dans la fraction GAGs ; ce résultat valide le test.

Le traitement par l'acide rétinoïque ne modifie pas de façon significative la synthèse des GAGs par les kératinocytes.

L'extrait peptidique d'avocat entraîne une augmentation significative de la synthèse des GAGS (+30%) (tableau 10).

**Tableau 10 - Synthèse des GAGs par des kératinocytes**

| | Incorporation 35S-sulfate (cpm) | pourcentage |
|---|---|---|
| Cellules contrôles | 11163 | |
| Calcium | 17753 | + 59%** |
| Acide rétinoïque | 13217 | +18% |
| Extrait Peptidique d'Avocat 0,005% MS | 14973 | + 34 %* |
| Extrait Peptidique d'Avocat 0,05% MS | 14833 | + 33% |

| | | |
|---|---|---|
| * p<0,05 ** p<0,01 - test de Dunett | | |

### 1.2.2 Effet sur la synthèse/libération de l'acide hyaluronique :

L'acide rétinoïque, référence du test, augmente significativement la quantité d'acide hyaluronique présente dans les surnageants de culture des kératinocytes. Sans effet sur la synthèse des GAGs, l'acide rétinoïque stimule spécifiquement la production d'acide hyaluronique.

L'extrait peptidique d'avocat à 0,005% augmente significativement la production d'acide hyaluronique (+30%) (tableau 11).

**Tableau 11 - Synthèse/Libération d'acide hyaluronique par des kératinocytes**

| | AH ng/ml | pourcentage |
|---|---|---|
| Cellules contrôles | 4684 | |
| Acide rétinoïque | 11503 | + 146% ** |
| Extrait Peptidique d'Avocat 0,005% MS | 6138 | + 31% * |
| Extrait Peptidique d'Avocat 0,05% MS | 4924 | +5% |

| | | |
|---|---|---|
| *p<0,05 ** p<0,01 - test de Dunett | | |

### II. Effet sur la néosynthèse des lipides épidermiques (Activité relipidante)

### II. 1 Matériel :

Des épidermes reconstruits à J5 [BIOalternatives] ont été cultivés dans les conditions suivantes :
- Epidermes contrôles (R) : milieu de différenciation [BIOalternatives],
- Epidermes témoins (T) : milieu de différenciation déplété,
- Extrait peptidique d'avocat, selon l'exemple 1, à 0,005% MS et 0,05% MS dans le milieu de différenciation déplété (application systémique).

### II.2 Méthode :

Les épidermes ont été placés en plaques 12 puits dans les conditions décrites ci-dessus (milieu de différenciation déplété ou non, contenant ou non l'extrait peptidique d'avocat).

Au bout de 24 heures, le milieu de culture a été renouvelé et supplémenté avec 0,75 µCi/ml de 14C-acétate [Amersham]. Les épidermes ont ensuite été incubés pendant 48 heures à 37°C et 5% de CO₂.

A la fin du temps de traitement, les épidermes ont été lavés au PBS [Invitrogen], dissociés de leurs nacelles et lysés par de l'acide perchlorique à 0,5 M sur glace.

Les lipides ont ensuite été extraits par une solution de méthanol/chloroforme (2:1). La radioactivité incorporée (correspondant aux lipides totaux) a alors été quantifiée par scintillation liquide. Après chromatographie sur couche mince, les lipides ont été analysés séparément par saisie directe de la radioactivité des différents spots et quantification à l'aide d'un phosphorimager de type Cyclone [Packard].

### II.3 Résultats :

### II.3.1 Effet sur la synthèse des lipides totaux :

Le milieu de différenciation complet (Epidermes R) induit une augmentation non significative de l'incorporation d'acétate dans les lipides totaux. L'extrait peptidique d'avocat n'affecte pas la synthèse des lipides totaux (tableau 12).

**Tableau 12 - synthèse des lipides totaux sur épidermes reconstruits**

| | Incorporation d'acétate (cpm) | Pourcentages |
|---|---|---|
| Epidermes T (milieu déplété) | 32171 | |
| Epidermes R (milieu de différenciation) | 41143 | + 28% (NS) |
| Extrait Peptidique d'Avocat 0,005% MS | 34705 | +11 % (NS) |
| Extrait Peptidique d'Avocat 0,05% MS | 35588 | +8% (NS) |

| | | |
|---|---|---|
| NS = non significatif | | |

### II.3.2 Analyse des profils de néosynthèse lipidique (Tableaux 12 et 13) :

Le milieu de différenciation complet (Epidermes R) induit une modification du profil des phospholipides avec notamment une augmentation des phospholipides (+24% par rapport aux épidermes T), du cholestérol sulfate (+13%). La quantité totale de céramides / cérébrosides n'est pas modifiée, par contre on note une modification importante des profils lipidiques obtenus : augmentation des céramides / cérébrosides les plus polaires (+38%) et diminution des céramides / cérébrosides les moins polaires (-16%) (Tableau 13). En ce qui concerne les lipides neutres, le milieu de différenciation complet induit une augmentation du cholestérol (+19%), des acides gras libres (+37%) et une diminution des acides gras estérifiés (-38%) (Tableau 14).

**Tableau 13 : Profils lipidiques dans le système « phospholipides + sphingolipides »**

| | Sphingomyéline | Phospholipides | Cholestérol sulfate | Céramides / Cérébrosides polaires | Céramides / Cérébrosides moins polaires |
|---|---|---|---|---|---|
| Epidermes T (milieu déplété) | 100 | 100 | 100 | 100 | 100 |
| Epidermes R (milieu de différenciation) | 105 | 124 | 113 | 138 | 84 |
| Extrait Peptidique d'Avocat 0,005% MIS | 90 | 104 | 101 | 130 | 86 |
| Extrait Peptidique d'Avocat 0,05% MS | 91 | 112 | 105 | 128 | 83 |

| | | | | | |
|---|---|---|---|---|---|
| (% du témoin T) | | | | | |

L'extrait peptidique d'avocat à 0,005% et 0,05% MS induit une augmentation des céramides / cérébrosides les plus polaires (+30% et +28% par rapport aux épidermes T), une diminution des céramides / cérébrosides les moins polaires (-14% et -17%) (Tableau 13) et à 0,05% une augmentation des acides gras libres (+32%) (Tableau 14). Ces effets sont similaires à ceux du milieu de différenciation complet.

**Tableau 14 : Profils lipidiques dans le système « lipides neutres + acides gras »**

| | Cholestérol | Acides gras libres | Acides gras estérifiés |
|---|---|---|---|
| Epidermes T (milieu déplété) | 100 | 100 | 100 |
| Epidermes R(milieu de différenciation) | 119 | 137 | 62 |
| Extrait Peptidique d'Avocat 0,005% MS | 112 | 103 | 93 |
| Extrait Peptidique d'Avocat 0,05% MS | 107 | 132 | 90 |

### (% du témoin T)

### III- Conclusion générale

Nous avons pu montrer un effet hydratant et relipidant de l'extrait peptidique d'avocat. En effet, l'extrait peptidique d'avocat stimule la synthèse des glycosaminoglycanes et de l'acide hyaluronique, qui sont impliqués dans le maintien d'un taux d'hydratation convenable de la peau. Et l'extrait peptidique d'avocat stimule la néosynthèse des lipides épidermiques, impliqués dans la fonction barrière de l'épiderme.

### Exemple 4 : formulations cosmétiques à base d'extrait peptidique d'avocat

| **EAU NETTOYANTE 1** | |
|---|---|
| **Matière première / Nom commercial** | **%** |
| Eau purifiée B4 | QSP 100 % |
| Biosaccharide gomme | De 1 à 5 % |
| Butylène glycol | De 1 à 5 % |
| Saponine purifiée | De 0 à 1 % |
| Eau de rose | De 0 à 1 % |
| Extrait peptidique d'avocat | De 0 à 5 % |
| Conservateurs | De 0 à 1 % |
| Allantoine | De 0 à 1 % |
| Acide citrique monohydrate | De 0 à 1 % |
| Trométhamine | De 0 à 1 % |

| **EAU NETTOYANTE 2** | |
|---|---|
| **Matière première / Nom commercial** | **%** |
| Capryloyl glycine | De 0 à 1 % |
| Lessive soude | De 0 à 1 % |
| Eau purifiée B4 | De 20 à 100 % |
| Séquestrant | De 0 à 1 % |
| Butylène glycol | De 1 à 5 % |
| Extrait peptidique d'avocat | De 0 à 5 % |
| Octanediol | De 0 à 1 % |
| PEG-32 | De 1 à 5 % |
| PEG-7 palmcocoate | De 1 à 5 % |
| Gluconate zinc | De 0 à 1 % |
| Acide citrique monohydrate | De 0 à 1 % |
| Eau purifiée B4 | QSP 100 % |
| Parfum | De 0 à 1 % |
| Poloxamer 184 | De 1 à 5 % |
| D.S.B. C SP | De 1 à 5 % |

| **EMULSION E/H** | |
|---|---|
| **Matière première / Nom commercial** | **%** |
| Isoparaffine liquide | De 5 à 20 % |
| Stéarate d'isocétyle | De 5 à 20 % |
| Hydrox stéarate Al - Mg | De 5 à 20 % |
| ABIL WE 09 | De 1 à 5 % |
| Glycérol | De 1 à 5 % |
| Huile vaseline épaisse | De 1 à 5 % |
| Zinc Oxyde micronisé | De 1 à 5 % |
| Butylène glycol | De 1 à 5 % |
| Extrait peptidique d'avocat | De 0 à 5 % |
| Isononyle isononanoate | De 1 à 5 % |
| Cire d'abeille blanche | De 1 à 5 % |
| Tartrate de sodium | De 1 à 5 % |
| Chlorure de sodium | De 0 à 5 % |
| Glycine | De 1 à 5 % |
| Octanediol | De 0 à 1 % |
| Cholestérol | De 0 à 1 % |
| Phytosphingosine | De 0 à 1 % |
| Acide tartrique | De 0 à 1 % |
| Eau purifiée B4 | QSP 100 % |

| **EMULSION H/E** | |
|---|---|
| **Matière première / Nom commercial** | **%** |
| Polydecène hydrogéné | De 5 à 20 % |
| Laurylglucoside-glystéarate | De 1 à 5 % |
| Dicaprylyle carbonate | De 1 à 5 % |
| Glycérol | De 5 à 20 % |
| Carbopol ETD 2020 | De 0 à 1 % |
| Gomme xanthane | De 0 à 1 % |
| Extrait peptidique d'avocat | De 0 à 5 % |
| Lessive soude | De 0 à 1 % |
| Conservateurs | De 0 à 1 % |
| Acide citrique monohydrate | De 0 à 1 % |
| Eau purifiée B4 | QSP 100 % |

| **HUILE** | |
|---|---|
| **Matière première / Nom commercial** | **%** |
| Solubilisant | De 0 à 1 % |
| Huile amande douce | De 5 à 20 % |
| Caprylate / caprate de coprah | QSP 100 % |
| Huile de macadamia raffinée | De 5 à 20 % |
| Caprylo caprate de glycérol | De 5 à 20 % |
| Alpha bisabolol nat | De 0 à 1 % |
| Alpha tocophérol | De 0 à 1 % |
| Extrait peptidique d'avocat | De 0 à 5 % |
| Conservateur | De 0 à 1 % |
| Ester | De 0 à 1 % |

| **LAIT** | |
|---|---|
| **Matière première / Nom commercial** | **%** |
| Huile amande douce | De 1 à 5 % |
| Huile mais | De 1 à 5 % |
| Acide stéarique | De 1 à 5 % |
| Alcool cétylique C16 C18 | De 0 à 1 % |
| Antimousse 70414 | De 0 à 1 % |
| Alcool laurique 11oe | De 1 à 5 % |
| Monolaurate PEG 300 | De 0 à 1 % |
| Monoléate de glycérol | De 0 à 1 % |
| Monosteéarate de glycérol | De 1 à 5 % |
| Extrait peptidique d'avocat | De 0 à 5 % |
| Conservateurs | De 0 à 1 % |
| Acide citrique monohydrate | De 0 à 1 % |
| Citrate trisodique | De 0 à 1 % |
| Eau purifiée | QSP 100 % |
| Parfum | De 0 à 1 % |
| Huile arachide | De 1 à 5 % |
| Huile palmiste hydrogénée | De 1 à 5 % |

| **MOUSSANT** | |
|---|---|
| **Matière première / Nom commercial** | **%** |
| Eau purifiée B4 | QSP 100 % |
| Lauroamphoacétate | De 5 à 20 % |
| Cocoglucoside | De 5 à 20 % |
| Oronal LCG | De 5 à 20 % |
| Hydriosul KMG 30 (2) | De 5 à 20 % |
| Distéarate de PEG 6000 | De 1 à 5 % |
| Conservateur | De 1 à 5 % |
| Extrait peptidique d'avocat | De 0 à 5 % |
| Extrait camomille | De 1 à 5 % |
| Acide citrique monohydrate | De 0 à 1 % |
| Séquestrant | De 0 à 1 % |
| Cocoproteine ble | De 0 à 1 % |
| Parfum mustiti 11/1 | De 0 à 1 % |
| Lessive soude | De 0 à 1 % |

| **SPRAY** | |
|---|---|
| **Matière première / Nom commercial** | **%** |
| Eau purifiée b4 | QSP 100 % |
| Trilaureth-4 phosphate | De 1 à 5 % |
| Dicaprylyl carbonate | De 1 à 5 % |
| Butylène glycol | De 1 à 5 % |
| Erythrityl ester | De 1 à 5 % |
| Huile vaseline fluide | De 1 à 5 % |
| Beurre de karité (liquide) | De 0 à 1 % |
| Jojoba pure | De 0 à 1 % |
| Conservateurs | De 0 à 1 % |
| Extrait peptidique d'avocat | De 0 à 5 % |
| Lessive soude | De 0 à 1 % |
| Parfum mustiti 10/3 | De 0 à 1 % |
| Gomme xanthane | De 0 à 1 % |
| Carbopol 981 NF | De 0 à 1 % |
| Séquestrant | De 0 à 1 % |
| Acide citrique monohydrate | De 0 à 1 % |

## Revendications

1. Extrait peptidique d'avocat, qui comprend 2 à 10% en poids d'azote alpha-aminé par rapport au poids de la matière sèche de l'extrait peptidique, et **caractérisé en ce qu'**au moins 50% des peptides de l'extrait sont constitués de 10 à 30 acides aminés, pour son utilisation dans le traitement ou la prévention du prurit interne dans un médicament ou une composition dermatologique comprenant ledit extrait et un excipient approprié.

2. Extrait peptidique d'avocat pour son utilisation selon la revendication 1, **caractérisé en ce qu'**il est susceptible d'être obtenu par un procédé comprenant les étapes suivantes :
- obtention d'un tourteau d'avocat, avantageusement du fruit de l'avocat, par séchage puis extraction de l'huile ; ensuite
- cryobroyage et délipidation complète dudit tourteau puis décantation, centrifugation et récupéreration du gâteau ; puis
- première hydrolyse en présence de glucanases, suivie d'une centrifugation et de l'élimination de la fraction soluble ;
- seconde hydrolyse en présence d'une ou plusieurs protéases, suivie d'une centrifugation et de l'élimination du culot ; ensuite
- concentration de la phase peptidique par nanofiltration et le cas échéant, décoloration en présence de charbon actif ; suivie
- d'une filtration simple (10 µm) puis d'une ultrafiltration (seuil de coupure de 10 kD) ; enfin
- le cas échéant, ajout de conservateur, microfiltration stérilisante finale (0,2 µm) et conditionnement.

3. Extrait peptidique d'avocat pour son utilisation selon les revendications 1 ou 2, **caractérisé en ce qu'**il présente la composition suivante en acides aminés, en pourcentage en poids par rapport au poids total d'acides aminés :
| | |
|---|---|
| Alanine | 6,4 - 7,8 |
| Arginine | 4,7 - 5,7 |
| Acide aspartique | 10,3 - 12,7 |
| Cystine-cystéine | 2,9 - 3,5 |
| Acide glutamique | 13,0 - 15,8 |
| Glycine | 5,3 - 6,5 |
| Histidine | 2,2 - 2,6 |
| Isoleucine | 4,8 - 5,8 |
| Leucine | 7,6 - 9,4 |
| Lysine | 3,0 - 3,8 |
| Méthionine | 1,2 - 1,6 |
| Phénylalanine | 4,7 - 5,7 |
| Proline | 4,1 - 5,2 |
| Sérine | 5,5 - 6,7 |
| Thréonine | 4,6 - 5,6 |
| Tyrosine | 3,6 - 4,4 |
| Valine | 5,8 - 7,2 |

4. Extrait peptidique d'avocat pour son utilisation selon l'une quelconque des revendications précédentes, dans laquelle ledit prurit est choisi dans le groupe constitué par le prurit gravidique, le prurit *sine materia* le prurit du nouveau-né et le prurit d'origine médicamenteuse, dans un médicament ou une composition dermatologique comprenant ledit extrait et un excipient approprié.

5. Extrait peptidique d'avocat pour son utilisation selon la revendication 4, **caractérisé en ce que** le prurit gravidique est choisi dans le groupe constitué par la cholestase gravidique et le prurigos gravidique.

6. Extrait peptidique d'avocat pour son utilisation selon la revendication 4, **caractérisé en ce que** le prurit *sine materia* est choisi dans le groupe constitué par le prurit psychogène et le prurit sénile.

7. Extrait peptidique d'avocat pour son utilisation selon l'une quelconque des revendications précédentes, **caractérisé en ce qu'**il présente en outre une activité hydratante et relipidante.

8. Extrait peptidique d'avocat pour son utilisation selon l'une quelconque des revendications précédentes, **caractérisé en ce que** le médicament ou la composition dermatologique comprend 0,01 à 20% en poids sec d'extrait peptidique d'avocat, par rapport au poids total dudit médicament.

9. Extrait peptidique d'avocat pour son utilisation selon l'une quelconque des revendications précédentes, caractérisé en que le médicament ou la composition dermatologique comprend au moins un agent anti-irritant et/ou apaisant et/ou cicatrisant et/ou anti-vieillissement et/ou hydratant.

10. Extrait peptidique d'avocat pour son utilisation selon la revendication 9, **caractérisé en ce que** l'agent anti-irritant et/ou apaisant et/ou cicatrisant et/ou anti-vieillissement et/ou hydratant est choisi dans le groupe constitué par la glycine, les sucres et/ou peptides végétaux, le lupéol, les oxazolines, les oxazolidines, l'acide lipoïque, l'alpha bisabolol, les dérivés de réglisse, l'enoxolone, l'ectoïne, les furanes d'avocat, les extraits de Centella asiatica, la caféine, le rétinol, le rétinal, l'acide rétinoïque, l'oxyde de zinc, le magnésium, le silicium, le cuivre, le zinc, le manganèse, le sélénium, l'acide hyaluronique, l'acide azélaïque et ses sels ou esters, l'acide salicylique et ses dérivés, l'alpha hydroxyacide (AHA), les esters d'AHA, l'acide pyrrolidone carboxylique et dérivés, les céramides, le cholesterol, le squalane, les phospholipides, le beta carotène, la vitamine A, la vitamine E, la vitamine C, la vitamine B3, la vitamine B5, la vitamine B6, le peroxyde de benzoyle, l'urée, la coenzyme Q10, la glucosamine et ses sels, la N-acétyl glucosamine, les eaux de source ou thermales, les peptides de soja et l'arabinogalactane.

## Patentansprüche

1. Avocado-Peptidextrakt, der 2 bis 10 Gew.% alpha-Aminostickstoff mit Bezug auf das Gewicht der Trockenmasse des Peptidextrakts umfasst, und **dadurch gekennzeichnet, dass** mindestens 50 % der Peptide des Extrakts aus 10 bis 30 Aminosäuren bestehen, für seine Verwendung bei der Behandlung oder der Prävention des internen Juckreizes in einem Arzneimittel oder einer dermatologischen Zusammensetzung, umfassend den Extrakt und einen geeigneten Trägerstoff.

2. Avocado-Peptidextrakt für seine Verwendung nach Anspruch 1, **dadurch gekennzeichnet, dass** er durch ein Verfahren mit den folgenden Schritten erhalten werden kann:
- Erhalten eines Avocado-Kuchens, vorteilhafterweise von der Avocadofrucht, durch Trocknen, dann durch Extrahieren des Öls, dann
- Kaltmahlen und vollständiges Entfetten des Kuchens, dann Dekantieren, Zentrifugieren und Wiedergewinnen des Kuchens; dann
- erste Hydrolyse in Anwesenheit von Glucanasen, gefolgt von einer Zentrifugierung und der Beseitigung der löslichen Fraktion;
- zweite Hydrolyse in Anwesenheit von einer oder von mehreren Proteasen, gefolgt von einer Zentrifugierung und der Beseitigung des Pellets; dann
- Konzentrieren der Peptidphase durch Nanofiltern und gegebenenfalls Entfärben in Anwesenheit von Aktivkohle; gefolgt von
- einer einfachen Filterung (10 µm), dann einer Ultrafilterung (Ausschlussgrenze 10 kD); dann
- gegebenenfalls Zugabe eines Konservierungsstoffs, endgültige sterilisierende Mikrofilterung (0,2 µm) und Verpackung.

3. Avocado-Peptidextrakt für seine Verwendung nach Anspruch 1 oder 2, **dadurch gekennzeichnet, dass** er die folgende Zusammensetzung aus Aminosäuren in Gewichtsprozentsatz mit Bezug auf das Gesamtgewicht der Aminosäuren aufweist:
| | |
|---|---|
| Alanin | 6,4 - 7,8 |
| Arginin | 4,7 - 5,7 |
| Asparaginsäure | 10,3 - 12,7 |
| Cystin-Cystein | 2,9 - 3,5 |
| Glutaminsäure | 13,0 - 15,8 |
| Glycin | 5,3 - 6,5 |
| Histidin | 2,2 - 2,6 |
| Isoleucin | 4,8 - 5,8 |
| Leucin | 7,6 - 9,4 |
| Lysin | 3,0 - 3,8 |
| Methionin | 1,2 - 1,6 |
| Phenylalanin | 4,7 - 5,7 |
| Prolin | 4,1 - 5,2 |
| Serin | 5,5 - 6,7 |
| Threonin | 4,6 - 5,6 |
| Tyrosin | 3, 6 - 4,4 |
| Valin | 5, 8 - 7,2 |

4. Avocado-Peptidextrakt für seine Verwendung nach einem beliebigen der vorhergehenden Ansprüche, wobei der Juckreiz ausgewählt ist aus der Gruppe, bestehend aus Schwangerschafts-Juckreiz, *Pruritus sine materia,* Neugeborenen-Juckreiz und medikamentösem Juckreiz, in einem Arzneimittel oder einer dermatologischen Zusammensetzung, umfassend den Extrakt und einen geeigneten Trägerstoff.

5. Avocado-Peptidextrakt für seine Verwendung nach Anspruch 4, **dadurch gekennzeichnet, dass** der Schwangerschafts-Juckreiz ausgewählt ist aus der Gruppe, bestehend aus der Schwangerschafts-Cholestase oder den Schwangerschafts-Prurigos.

6. Avocado-Peptidextrakt für seine Verwendung nach Anspruch 4, **dadurch gekennzeichnet, dass** der *Pruritus sine materia* ausgewählt ist aus der Gruppe, bestehend aus dem psychogenen Juckreiz und dem senilen Juckreiz.

7. Avocado-Peptidextrakt für seine Verwendung nach einem beliebigen der vorhergehenden Ansprüche, **dadurch gekennzeichnet, dass** er außerdem eine feuchtigkeitsspendende und rückfettende Aktivität aufweist.

8. Avocado-Peptidextrakt für seine Verwendung nach einem beliebigen der vorhergehenden Ansprüche, **dadurch gekennzeichnet, dass** das Arzneimittel oder die dermatologische Zusammensetzung 0,01 bis 20 % Trockengewicht Avocado-Peptidextrakt mit Bezug auf das Gesamtgewicht des Arzneimittels umfasst.

9. Avocado-Peptidextrakt für seine Verwendung nach einem beliebigen der vorhergehenden Ansprüche, **dadurch gekennzeichnet, dass** das Arzneimittel oder die dermatologische Zusammensetzung mindestens ein reizvermeidendes, und/oder lindernd wirkendes und/oder wundheilendes und/oder Anti-Aging und/oder feuchtigkeitsspendendes Mittel umfasst.

10. Avocado-Peptidextrakt für seine Verwendung nach Anspruch 9, **dadurch gekennzeichnet, dass** das reizvermeidende und/oder lindernd wirkende und/oder wundheilende und/oder Anti-Aging und/oder feuchtigkeitsspendende Mittel ausgewählt ist aus der Gruppe, bestehend aus Glycin, pflanzlichen Zuckern und/oder Peptiden, Lupeol, Oxazolinen, Oxazolidinen, Liponsäure, alpha-Bisabolol, Derivaten des echten Süßholzes, Enoxolon, Ectoin, Avocadofuranen, Extrakten von *Centella asiatica,* Koffein, Retinol, Retinal, Retinsäure, Zinkoxid, Magnesium, Silizium, Kupfer, Zink, Mangan und Selen, Hyaluronsäure, Azelainsäure und ihren Salzen oder Estern, Salicylsäure und ihren Derivaten, Alphahydroxysäure (AHA), Estern von AHA, Carboxyl-Pyrrolidonsäure und ihren Derivaten, Cermaiden, Cholesterin, Squalan, Phospholipiden, Betacarotin, Vitamin A, Vitamin E, Vitamin C, Vitamin B3, Vitamin B5, Vitamin B6, Benzoylperoxid, Harnstoff, Coenzym Q10, Glucosamin und seinen Salzen, N-Acetyl-Glucosamin, Quell- oder Thermalwassern, Sojapeptiden und Arabinogalactan.

## Claims

1. Avocado peptide extract, which comprises 2% to 10% by weight of alpha-amino nitrogen in relation to the weight of the dry matter of the peptide extract, and **characterised in that** at least 50% of the peptides of the extract consist of 10 to 30 amino acids, for use in treating or preventing internal pruritus in a drug or a dermatological composition comprising said extract and a suitable excipient.

2. Avocado peptide extract for use according to claim 1, **characterised in that** it is obtainable by a method comprising the following steps:
- obtaining an avocado oil cake, advantageously from avocado fruit, through drying and extraction of the oil; followed by
- cryogrinding and complete delipidation of said oil cake followed by decantation, centrifugation and collection of the oil cake; then
- first hydrolysis in the presence of glucanases, followed by centrifugation and elimination of the soluble fraction;
- second hydrolysis in the presence of one or more proteases, followed by centrifugation and elimination of the pellet; followed by
- concentration of the peptide phase by nanofiltration and, if applicable, discolouration in the presence of activated carbon; followed by
- simple filtration (10 µm) followed by ultrafiltration (10 kDa cutoff); and finally
- if applicable, addition of preservative, final sterilising microfiltration (0.2 µm) and packaging.

3. Avocado peptide extract for use according to claims 1 or 2, **characterised in that** it has the following amino acid composition, as a weight percentage in relation to the total weight of the amino acids:
| | |
|---|---|
| Alanine | 6.4-7.8 |
| Arginine | 4.7-5.7 |
| Aspartic acid | 10.3-12.7 |
| Cystine-cysteine | 2.9-3.5 |
| Glutamic acid | 13.0-15.8 |
| Glycine | 5.3-6.5 |
| Histidine | 2.2-2.6 |
| Isoleucine | 4.8-5.8 |
| Leucine | 7.6-9.4 |
| Lysine | 3.0-3.8 |
| Methionine | 1.2-1.6 |
| Phenylalanine | 4.7-5.7 |
| Proline | 4.1-5.2 |
| Serine | 5.5-6.7 |
| Threonine | 4.6-5.6 |
| Tyrosine | 3.6-4.4 |
| Valine | 5.8-7.2 |

4. Avocado peptide extract for use according to any one of the preceding claims, wherein said pruritus is selected from the group comprising pruritus gravidarum, pruritus *sine materia,* neonatal pruritus and drug-related pruritus, in a drug or a dermatological composition comprising said extract and a suitable excipient.

5. Avocado peptide extract for use according to claim 4, **characterised in that** the pruritus gravidarum is selected from the group comprising obstetric cholestasis and prurigo gravidarum.

6. Avocado peptide extract for use according to claim 4, **characterised in that** the pruritus *sine materia* is selected from the group comprising psychogenic pruritus and senile pruritus.

7. Avocado peptide extract for use according to any one of the preceding claims, **characterised in that** it further has a hydrating and refatting activity.

8. Avocado peptide extract for use according to any one of the preceding claims, **characterised in that** the drug or dermatological composition comprises 0.01% to 20% in dry weight of the avocado peptide extract in relation to the total weight of said drug.

9. Avocado peptide extract for use according to any one of the preceding claims, **characterised in that** the drug or dermatological composition further comprises at least one anti-irritant and/or soothing and/or cicatrising and/or anti-aging and/or hydrating agent.

10. Avocado peptide extract for use according to claim 9, **characterised in that** the anti-irritant and/or soothing and/or cicatrising and/or anti-aging and/or hydrating agent is selected from the group comprising glycine, plant sugars and/or peptides, lupeol, oxazolines, oxazolidines, lipoic acid, alpha bisabolol, liquorice derivatives, enoxolone, ectoine, avocado furans, Centella asiatica extracts, caffeine, retinol, retinal, retinoic acid, zinc oxide, magnesium, silicon, copper, zinc, manganese, selenium, hyaluronic acid, azelaic acid and salts or esters thereof, salicylic acid and derivatives thereof, alpha hydroxy acid (AHA), AHA esters, pyrrolidone carboxylic acid and derivatives thereof, ceramides, cholesterol, squalane, phospholipids, beta carotene, vitamin A, vitamin E, vitamin C, vitamin B3, vitamin B5, vitamin B6, benzoyl peroxide, urea, coenzyme Q10, glucosamine and salts thereof, N-acetyl glucosamine, thermal or spring waters, soya peptides and arabinogalactan.
